# EUROPEAN PATENT APPLICATION

(11) **EP 1 997 903 A1**
(43) Date of publication of application: **03.12.2008**
(21) Application number: 08104177.4
(22) Date of filing: 30.05.2008
(51) Int. Cl.: C12Q 1/06

(54) **A method to discriminate and quantify Torulaspora delbrueckii in mixture with Kluyveromyces thermotolerans and Saccharomyces cerevisiae.**

(30) Priority: 31.05.2007 DK 200700796
(71) Applicant: Chr. Hansen A/S, 2970 Hørsholm (DK)
(72) Inventor: Stolpe, Eva, 2610, Rødovre (DK)
(74) Representative: Hagen, Klaus Bach

(57) **Abstract**

A method useful for the discrimination and quantification of *Torulaspora delbrueckii* (TD), which can be present in a mix together with *Kluyveromyces thermotolerans* (KT) and *Saccharomyces* in a starter culture or in cultures in general.

## Description

### FIELD OF THE INVENTION

The present invention relates to a method useful for the discrimination and quantification of *Torulaspora delbrueckii* (TD), which can be present in a mix together with *Kluyveromyces thermotolerans* (KT) and *Saccharomyces* in a starter cultures or in cultures in general.

### BACKGROUND ART

It is common to use yeasts in the wine industry. Typical yeasts used in wine production are from the yeast family *Saccharomycetaceae* (ascomycetous yeasts). Yeasts from the genus *Saccharomyces* [e.g. the specie *Saccharomyces cerevisiae* (SC)] are commonly used. Other used yeasts are from the same Saccharomycetaceae family but from other genera such as *Kluyveromyces* [e.g. the specie *Kluyveromyces thermotolerans* (KT)] and the genus *Torulaspora* [e.g. the specie *Torulaspora delbrueckii* (TD)].

The company Chr. Hansen, Denmark produces yeast starter cultures which contain *Saccharomyces, Kluyveromyces* and/or *Torulaspora.* Each of these cultures are manufactured separately, mixed in specific ratios and thereafter packaged and made ready for use in fermentation processes such as the preparation of wine. W02004/072271 (Chr. Hansen) describes such starter cultures.

For a commercially available starter culture it is important to know the specific amount/ratio of each yeast species in the final commercially available starter culture. This is in particular true for a wine starter culture comprising *Saccharomyces, Kluyveromyces* and/or *Torulaspora.*

The wine industry has established some standard tests to measure yeast amounts in wine cultures. The International Organisation of Vine and Wine (O.I.V) is a well-known organization within the wine industry. In the standard test described in the O.I.V. "Resolution OENO 16/2003" it is explained how to quantify yeast of different species of *Saccharomyces* according to a so-called lysine test. At the filing date of the present application the "Resolution OENO 16/2003" was freely available on the Internet.

In this standard test it is only described how to quantify amounts of *Saccharomyces* species in the wine starter culture. In other words, it is not described how to quantify amounts of non*-Saccharomyces* yeast such as *Kluyveromyces* and/or *Torulaspora.*

### SUMMARY OF THE INVENTION

The problem to be solved by the present invention is to provide a new improved assay that can discriminate *Saccharomyces* from non*-Saccharomyces* as present in mixtures (in products such as starter cultures, in bulk, or in must of wine) and quantify *Saccharomyces* and non*-Saccharomyces* species individually.

The solution is based on that the present inventors have identified that the special agar medium described herein is a selective medium where only *T. delbrueckii* (TD) is able to grow, i.e. *Saccharomyces cerevisiae* (SC), *Kluyveromyces thermotolerans* (KT) are not growing on this medium. Accordingly, by use of this special agar medium one can positively determine TD (cfu/g) directly in a mixed culture comprising SC and/or KT.

Accordingly, a first aspect of the invention relates to a method to discriminate and quantify *Torulaspora delbrueckii* (TD) in a mixed culture comprising *Saccharomyces cerevisiae* (SC) or SC and *Kluyveromyces thermotolerans* (KT) comprising:
i) Pour plating the mixed culture on dishes containing a TD medium comprising agar, wherein the agar in the TD medium is agar prepared from red seaweed belonging to the class of *Rhodophycea* and incubate at 25°C +/- 2°C to determine TD and quantify TD (cfu/g).

By using the TD medium as disclosed, it becomes now possible to accurately discriminate and quantify TD in a mixture of SC and KT since only TD grows under these conditions.

A second aspect of the invention is a diagnostic kit to discriminate and quantify *Torulaspora delbrueckii* (TD) in a mixed culture comprising *Saccharomyces cerevisiae* (SC) or SC and *Kluyveromyces thermotolerans* (KT) the kit comprising a TD medium comprising agar, wherein the agar in the TD medium is agar prepared from red seaweed belonging to the class of *Rhodophycea* and instructions to incubate at 25°C +/-2°C to determine TD and quantify TD (cfu/g).

Embodiment of the present invention is described below, by way of examples only.

### DETAILED DESCRIPTION OF THE INVENTION

It is a primary objective of this invention to provide an improved method for the discrimination and quantification of *Saccharomyces* and non*-Saccharomyces.*

Temperature and growth media are two parameters used to discriminate and quantify strains present in mixtures. By using these criteria one makes use of the characteristics of the specific strains, often on species level. These factors need to be discovered experimentally. There are three steps involved as explained below.

### Discrimination and quantification of Saccharomyces cerevisiae (SC)

SC in mixture with KT and TD can be selected by growing the SC on a rich medium at 37°C +/- 2°C. This allows growth on dishes of SC alone as KT and TD do not grow at +37°C +/- 2°C. Rich medium is a medium suitable for growth of virtually all yeast types and typically contains glucose, a nitrogen source, yeast extract and peptones, and other growth factors important for yeast. A rich medium can be a YGC medium as used in the described methods in the working examples herein.

### Discrimination and quantification of Kluyveromyces thermotolerans (KT)

### Temperature for growth of KT:

KT in mixture with SC and TD can be discriminated and quantified by using lysine medium as generally available in trade and incubating the dishes at 32°C +/- 0.5°C.

### Media for pour plating:

Lysine media is a synthetic medium for the isolation and enumeration of wild yeast encountered in brewing. A lysine medium is a medium containing lysine as the sole nitrogen source.

Typically a lysine media comprises glucose, lysine, trace elements (Zn, B, Mn), salts, amino acids, and mineral nutrition elements (Ca, N, P, K).

In the preferred embodiment the lysine media is the lysine media CM0191 from the company Oxoid, which is used in working examples herein.

### Discrimination and quantification of Torulaspora delbrueckii (TD)

TD in mixture with SC and KT can be discriminated and quantified by using the herein disclosed media and by incubating the dishes at +25°C +/- 2°C.
Preferably there is incubated for from 48 hours to 120 hours, more preferably for from 70 hours to 98 hours.

### TD Medium:

The standard test described in the O.I.V. "Resolution OENO 16/2003" (discussed in background section above) describes the general conditions needed to count different species of *Saccharomyces* strains according to the lysine test (see page 5) using an incubation temperature of +25°C. The type of agar is not specified in that reference. Further TD is not a *Saccharomyces* strain.

Several types of agar were tried by the inventors and did not result in a TD selective medium, where only TD grows. However, after several different tests the inventors identified the suitable TD selective medium discussed herein.

A specific type of agar from a specific supplier with the product name Bacteriological Agar-Agar (from the company So.Bi.Gel S.A) was identified to be a herein suitable TD selective medium.

This agar type comprises agar prepared from red seaweed belonging to the class of *Rhodophycea.*

More specifically this agar type comprises a mix of 2 polysaccharides, specified as agarose 1,4 linked 3,6 anhydro-L-Galactose and 1,3 linked D-Galactose, and agaropectine specified as agarose and ester-sulphate and D-glucoronic acid and piruvic acid.

As can be seen in table 3 of the working example herein only TD, and not SC and KT, is growing in this special agar medium.

Accordingly, a preferred embodiment is herein a method, wherein it is essentially only TD that grows on the TD medium at the conditions the first aspect, i.e. there are less than 10 detectable colonies (preferably no detectable colonies) of SC and KT after 72 hours incubation.

The TD medium may also comprise lysine, glucose and chloramphenicol.

### Commercially relevant uses of the method as described herein

In a preferred embodiment the method can be used to discriminate and quantify *Saccharomyces* (such as SC) and non*-Saccharomyces* cultures (such as KT and TD), as a quality control method by servicing laboratories for e.g. finished starter cultures with the purpose of checking whether the mixing of the cultures is done correctly and within set specifications.

In a preferred embodiment the method can be used to discriminate and quantify *Saccharomyces* and *non-Saccharomyces* cultures such as e.g. fermentation must samples during the fermentation of wines for wine producers. This will help the wine producer to control the wine production.

### EXAMPLES

### Example 1. Discrimination and quantification of mixed cultures containing SC, KT and TD

### Material and methods

### 1. Yeast strains

*Saccharomyces cerevisiae* (SC): CHCC 5754 with CBS 9050 number and commercially available from Chr. Hansen.
*Torulaspora delbrueckii* (TD) with deposition number CBS 9047
*Kluyveromyces thermotolerans* (KT) with deposition number CBS 9048

### 2. Chemicals for media compositions.

Yeast extract (Oxoid no. L21), Lysine-medium (Oxoid no. CM0191), Lactic acid 10% ampoule (Oxoid no. SR021H), Potassium lactate 50% ampoule (Oxoid no. SR033H) NaCL (Merck no. 6404), Nitrogen base (DIFCO 0392-15), D (+) Galactose (Merck 2.04062.), Bacteriological Aqar-Aqar (SO-BI-GEL), L-Lysine monohydrochloride (FLUKA 62930), D (+) Glucose-monohydrate (Merck 1.08342), Chloramphenicol (Merck 1.02366.0050), Glass distilled water, 1 N NaOH.

### 3. Dilution medium.

To make sure that the data reflect the dependence of media as selection criteria only, the SC, TD and KT cells are kept (diluted) in 0.9 % NaCl (rest water) before pour plating. In this salt solution there are thus no nutrients and none of the tested strains can grow.

### 4. Media

| YGC medium | |
|---|---|
| D-glucose-monohydrate | 20 g/l |
| Agar-Agar | 15 g/l |
| Yeast extracts | 5.0 g/l |
| Chloramphenicol | 0.1 g/l |
| Distilled water up to | 1 l |

YGC medium (composition and preparation according to DK-BIRA-QAm-002)

| Lysine medium | |
|---|---|
| Lysine medium CM0191 | 66 g/l |
| Potassium lactate 50% | 10 ml/l |
| Lactic acid 10% | 1 ml/l |
| Glass distilled water up to | 1 l |

Suspend 6.6 g of Lysine medium in 100 ml of sterile distilled water. 1 ml of a 50 % Potassium lactate solution is added. Heat the suspension to boiling point and leave it boiling for approximately 10 min. Cool down to 45°C and add 0.1 ml of lactic acid from a 10% solution (pH obtained is 4.8 +/- 0.2). The media is prepared the same day as it is used.

| *Torulaspora delbrueckii* TD medium | |
|---|---|
| Bacteriological Agar-Agar | 15 g/l |
| Lysine | 5 g/l |
| Glucose | 1 g/l |
| Chloramphenicol | 0.1 g/l |
| Glass distilled water up to | 1 I |
| Adjust pH to 6.8 | |

Weigh out 5 x 3 g of Agar-Agar in five bottles. Lysine, glucose and Chloramphenicol is mixed with glass distilled water up to 1 I. pH is adjusted to 6.8 with 1 N NaOH. The Lysine solution is poured into the bottles containing the agar (up to 200 ml/bottle). The medium is autoclaved at 121°C, 15 min. Use the medium just after autoclaving or store it at + 5°C until use.

### 5. Equipment

Welding machine, Autoclave, Incubator 25°C, 30°C and 37°C, Bottles of 250 ml Balance operating at +/- 0.001 g, Autoclave or water boiler bath, operating at +/-1°C
Water bath, operating +/-1°C, Petri dishes, sterile, 9 mm, Pipettes, Sterile pipettes tips, Clean-bench, Filter 0.45 my.

### RESULTS

### Discrimination and quantification of SC

Pure culture of SC is tested on YGC medium, on Lysine medium and on TD medium.

Total CFU/ g of *S. cerevisiae* (SC) is measured on YGC-agar; pour plating, 48 hours (2 days) aerobic incubation at 37°C. Only colonies of *S. cerevisiae* (SC) appeared on the plates, *non-Saccharomyces* yeast is not able to grow at 37°C.

### Results

**Table 1. Total cell cfu/g count of Saccharomyces cerevisiae (SC) after 48 hours. SC is not growing on the other two media (lysine and TD medium).**

| Replicate no. | YGC Medium + 37°C | Lysine medium +32°C | TD medium +25 °C |
|---|---|---|---|
| 1 | 1.7 × 10¹⁰ | - | - |
| 2 | 1.6 × 10¹⁰ | - | - |
| 3 | 1.9 × 10¹⁰ | - | - |
| 4 | 1,6 × 10¹⁰ | - | - |
| 5 | 1,7 × 10¹⁰ | - | - |

| | | | |
|---|---|---|---|
| "-" Means no detectable growth | | | |

### Conclusion:

SC is selectively growing on YGC medium when incubated at 37°C.
KT and TD are not growing on YGC medium at 37°C.

### Discrimination of KT and quantification of KT

Lysine medium CM0191 incubated at + 32°C incubation for 72 hours (three days).

Pure culture of KT was tested on YGC medium, on lysine medium and on TD medium.

Total CFU/ g of K. *thermotolerans* (KT) is measured on Lysine-agar by pour plate method and incubate for 72 hours (3 days) of aerobic incubation at +32°C. The temperature inhibits the growth of T. *delbrueckii* (TD). SC cannot grow on lysine as sole nitrogen source. By choosing lysine as a nitrogen source, one deselects SC. Their colonies will appear as pinpoint colonies after 72 hours to 96 hours (3 to 4 days) of incubation at 32°C *K. thermotolerans* (KT) will obtain a colony size of 1-2 mm.

### Results

**Table 2. Total cell cfu/g count of Kluyveromyces thermotolerans (KT) after 72 hours (3 days).**

| Replicate no. | YGC medium 37°C | Lysine medium + 32°C | TD medium + 25°C |
|---|---|---|---|
| 1 | - | 2.9 x 10⁹ | - |
| 2 | - | 4.2 x 10⁹ | - |
| 3 | - | 4.0 x 10⁹ | - |
| 4 | - | 4.2 x 10⁹ | - |
| 5 | - | 4.7 x 10⁹ | - |

### Conclusion

Selective growth of KT alone with the chosen lysine medium CM0191. KT cannot grow on YGC medium and cannot grow on TD medium.

### Discrimination and quantification for Torulaspora delbrueckii (TD)

### Working example of the different media composition types.

The reason for using reference [1] is that it is known that *Saccharomyces* species grow on such a medium. The inventor tried Lysine medium as specified in reference [1]. However, by using SC, KT and TD in mixtures, the result was that TD was not really growing. SC and KT had small colonies. Typically the colonies of TD were too small, indicating that this media is not suitable for growth of TD and therefore creates doubt for the discrimination and quantification of non- *Saccharomyces* species.

As a result, the inventor was forced to experiment with the composition of the medium using reference [1] as inspiration. Some ingredients were removed and salt and different quantities of sugar were checked for the possibility of discrimination and enumeration of TD. The indicator was also taken out (Bromocresol purple) with disappointing results.

### Agar types.

The inventor then experimented with different agar types such as; Bactoagar (Difco) 214010 and Bacteriological Agar-Agar from the SO.Bi.Gel company.

This agar is prepared from red seaweed, class *Rhodophycea* and is a mixture of two polysaccharides, agarose (1,4 linked 3,6 anhydro-L-Galactose and 1,3 linked D-Galactose) and an agaropectin.

SC, TD and KT were prepared separately and diluted in 0.9% NaCl. Thereafter SC was pour plated, TD was pour plated and KT was pour plated on the same plate (dish), using the same concentration of cells of all three (10⁷ cfu/g).

**Table 3: SC, Kt and TD counts using Bactoagar and Agar-Agar in two salt concentrations 0.25% and 0.50%. Incubation time from 72 hours (3 days) to 96 hours (4 days).**

| Yeast type | OIV + 0.25% salt - Bacto agar +25°C | OIV + 0.25% salt - Agar- Agar +25°C | OIV +0.5% salt Bacto agar + 25°C | OIV + 0.5% salt Agar-Agar + 25°C |
|---|---|---|---|---|
| SC | - | - | - | - |
| KT | 1.7 x 10¹⁰ | - | 1.4 x 10¹⁰ | - |
| TD | 2.1 x 10¹⁰ | 1.7 x 10¹⁰ | 1.9 x 10¹⁰ | 1.5 x 10¹⁰ |
| SC+KT+TD | 2.5 x 10¹⁰ | 1.5 x 10¹⁰ | 2 x 10¹⁰ | 1.3 x 10¹⁰ |

| | | | | |
|---|---|---|---|---|
| OIV = reference [1] lysine media page 5. - = none detectable colonies | | | | |

By using Bactoagar in the media, the counts as mentioned in the table 3 (2.5 x 10¹⁰) indicate that there is more than one yeast species (2.5 x 10¹⁰ > TD 2.1 x 10¹⁰) present on the plate.

Using Agar-Agar, the TD counts of 1.5 x10¹⁰ (both for the two salt concentrations) is closer to the pure isolate pour plated. (See table 3 and compare 1.7 x 10¹⁰ with 1.5 x 10¹⁰ and 1.5 x 10¹⁰ with 1.3 x 10¹⁰).

### Conclusion.

Bacteriological Agar-Agar as a specific choice of agar in the media shows that it helps to discriminate TD from KT and SC and discriminates better than Bactoagar. Salt in two concentrations did not seem to have any influence in the discrimination of the strains.

### Working example with the unique type of agar.

*Torulaspora delbrueckii* (TD) medium. Total CFU/g *T. delbrueckii* (TD) is measured on *Torulaspora delbrueckii* medium by pour plating, 96 hours (4 days) of aerobic incubation at 25°C.
The medium is a selective medium where only *T. delbrueckii* (TD) is able to grow.
The selection of Bacteriological Agar-Agar from So.Bi.Gel is important for the results of the selective pressure on the three different strains.

### Results.

**Table 4. Total cell cfu/g count of Torulaspora delbrueckii (TD) after 96 hours (4 days).**

| Replicate no. | YGC medium +37°C | Lysine medium +32°C | TD medium +25°C |
|---|---|---|---|
| 1 | - | - | 1.9×10¹⁰ |
| 2 | - | - | 1.9×10¹⁰ |
| 3 | - | - | 2.0×10¹⁰ |
| 4 | - | - | 1.9×10¹⁰ |
| 5 | - | - | 2.1×10¹⁰ |

### Conclusion.

TD appears alone on the selected TD medium at 25°C.
No growth of SC and KT on TD medium at selected temperature.
TD cannot grow on YGC medium at 37°C and cannot grow on lysine medium at 32°C.

### REFERENCES

1. Resolution OENO 16/2003,
   http://news.reseau-
   concept.net/images/oiv_uk/Client/Resolution_Oeno_EN_2003_16.pdf

## Claims

1. A method to discriminate and quantify *Torulaspora delbrueckii* (TD) in a mixed culture comprising *Saccharomyces cerevisiae* (SC) or SC and *Kluyveromyces thermotolerans* (KT) comprising:
i) Pour plating the mixed culture on dishes containing a TD medium comprising agar, wherein the agar in the TD medium is agar prepared from red seaweed belonging to the class of *Rhodophycea* and incubate at 25°C +/- 2°C to determine TD and quantify TD (cfu/g).

2. The method of claim 1, wherein the agar in the TD medium is agar-agar and wherein the agar-agar comprises a mix of 2 polysaccharides, specified as agarose 1,4 linked 3,6 anhydro-L-Galactose and 1,3 linked D-Galactose, and agaropectine specified as agarose and ester-sulphate and D-glucoronic acid and piruvic acid.

3. The method of any of claims 1 to 2, wherein the mixed culture also comprises *Kluyveromyces thermotolerans* (KT), i.e. it comprises TD, SC and KT.

4. The method of claim 3, wherein it is essentially only TD that grows according to claim 1, i.e. there are less than 10 detectable colonies (preferably none detectable colonies) of SC and KT after 72 hours incubation.

5. The method of any of claims 1 to 4, wherein the amount of *Saccharomyces cerevisiae* (SC) is additionally quantified by pour plating the mixed culture on dishes containing a rich medium wherein the rich medium is selected from the group of yeast extracts/media such as YGC media and wherein the dishes are incubated at +37°C +/- 2°C to determine SC and quantify SC (cfu/g).

6. The method of any of claims 3 to 5, wherein the amount of KT is additionally quantified by pour plating the mixed culture on dishes containing a lysine medium and incubating the dishes at a temperature of +32°C +/- 0.5°C to determine KT and quantify KT (cfu/g).

7. The method any of claims 1 to 6, wherein the method is used to discriminate and quantify *Saccharomyces* and non*-Saccharomyces* cultures as quality control method by laboratories for finished starter cultures with the purpose of checking whether the mixing is done correctly.

8. The method of any of claims 1 to 6, wherein the method is used to discriminate and quantify *Saccharomyces* and non*-Saccharomyces* cultures such as e.g. fermentation must samples during the fermentation of wines for wine producers.

9. The method of any of the preceding claims, wherein there in the method of claim 1 is incubated for from 48 hours to 120 hours, more preferably for from 70 hours to 98 hours.

10. A diagnostic kit to discriminate and quantify *Torulaspora delbrueckii* (TD) in a mixed culture comprising *Saccharomyces cerevisiae* (SC) or SC and *Kluyveromyces thermotolerans* (KT) the kit comprising a TD medium comprising agar, wherein the agar in the TD medium is agar prepared from red seaweed belonging to the class of *Rhodophycea* and instructions to incubate at 25°C +/- 2°C to determine TD and quantify TD (cfu/g).
